# DEMANDE DE BREVET EUROPEEN

(11) **EP 0 881 283 A1**
(43) Date de publication de la demande: **02.12.1998**
(21) Numéro de dépôt: 97201628.1
(22) Date de dépôt: 31.05.1997
(51) Int. Cl.: C12N 1/20, C12P 19/08, C12N 9/10, A23L 1/054, A61K 7/00

(54) **Production de dextrane**

(71) Demandeur: SOCIETE DES PRODUITS NESTLE S.A., 1800 Vevey (CH)
(72) Inventeur: Schmid, Daniel, 1005 Lausanne (CH); D'Amico, Nicola, 1436 Treycovagnes (CH); Eyer, Kurt, 3600 Thun (CH); Aebischer, Jürg, 3097 Liebefeld (CH); De Maleprade, Dominique, 1800 Vevey (CH); Reniero, Roberto, 1801 Le Mont-Pelerin (CH); Neeser, Jean-Richard, 1073 Savigny (CH); Lesens, Corinne, 60000-Beauvais (FR)
(74) Mandataire: Wavre, Claude-Alain

(57) **Abrégé**

L'invention concerne une souche bactérienne produisant du dextrane, un procédé de production de dextrane, un procédé de production d'un additif contenant une enzyme impliquée dans la biosynthèse du dextrane ainsi que l'utilisation de ce dextrane, de cette enzyme et/ou d'une souche productrice de ce dextrane et de cette enzyme dans la fabrication d'une composition alimentaire ou cosmétique.

## Description

La présente invention a pour objet une souche bactérienne produisant du dextrane, un procédé de production de dextrane, un procédé de production d'un additif contenant une enzyme impliquée dans la biosynthèse du dextrane ainsi que l'utilisation de ce dextrane, de cette enzyme et/ou d'une souche productrice de ce dextrane et de cette enzyme dans la fabrication d'une composition alimentaire ou cosmétique.

### ETAT DE LA TECHNIQUE

Le dextrane est un polysaccharide formé d'unités de glucose dont l'allongement des chaines est catalysé par la dextrane-sucrase. La biosynthèse du dextrane a été mise en évidence chez de nombreuses bactéries, notamment chez les *Streptococcus mutans*, chez les *Leuconostoc mesenteroides ssp mesenteroides* et chez les *Leuconostoc mesenteroides ssp. dextranicum*. Les *Leuconostoc* produisent l'enzyme, la dextrane-sucrase, et la secrètent dans le milieu de culture en présence de saccharose. Cette enzyme, la dextrane-sucrase, synthétise alors le dextrane à partir du substrat, le saccharose. Le dextrane trouve son application dans plusieurs domaines. Il est notamment utilisé en biochimie comme support pour la chromatographie par filtration sur gel du type Sephadex. De plus, dans le domaine thérapeutique, il est utilisé comme succédané du plasma sanguin (Biochimie générale - J.H. WEIL - Masson 6ème edt° - 1990 - p171).

De plus, le dextrane synthétisé par une souche de *Leuconostoc dextranicum trouve* son application dans l'industrie alimentaire pour la texturation de compositions alimentaires, tels que les yogourts, les crèmes dessert, les boissons à base de lait et les sauces à salade. Ainsi, EP 0363633 met en évidence la synthèse de dextrane par une souche de *Leuconostoc dextranicum* et en particulier par la souche de *Leuconostoc dextranicum* NRRL-B-18242. Ce document décrit notamment une composition contenant le dextrane synthétisé par cette bactérie et l'utilisation de cette composition dans le domaine alimentaire.

Par ailleurs, la taxonomie des souches bactériennes du genre *Leuconostoc* a été plusieurs fois remaniée.

Ainsi, Garvie et al. (International Journal of Systematic Bacteriology, 118-119, 1983) décrivent la taxonomie des souches bactériennes du genre *Leuconostoc* établie selon un critère d'homologie au niveau de l'acide désoxyribonucléique (ADN). Les bactéries, précédemment classées comme des *Leuconostoc mesenteroides*, des *Leuconostoc dextranicum* et des *Leuconostoc cremoris*, bien que de phénotype différent, présentent une très grande homologie au niveau de leur ADN. Aussi, selon cette taxonomie, ces bactéries sont des sous-espèces de *Leuconostoc mesenteroides* et sont dénomées respectivement; *Leuconostoc mesenteroides* ssp. *mesenteroides*, *Leuconostoc mesenteroides ssp*. *dextranicum* et *Leuconostoc mesenteroides ssp. cremoris*.

De plus, J.B. Milliere et al. (Journal of Applied Bacteriology, 67, 529-542, 1989) décrivent une analyse taxonomique, effectuée sur 81 souches du genre *Leuconostoc*, dont 11 souches de *Leuconostoc*/*mesenteroides ssp. cremoris*. Cette analyse s'appuie sur la taxonomie établie par Garvie et al. et est basée, notamment, sur les critères suivants: capacité de ces souches à fermenter les différents sucres, capacité de ces souches à utiliser le citrate et capacité de ces souches à produire du dextrane. Ce document constate le fait qu'une souche de *Leuconostoc mesenteroides ssp. cremoris* ne synthétise pas de dextrane. De plus, une telle souche se distingue et se définit par le fait qu'elle ne fermente pas les pentoses.

Jusqu'à ce jour, aucune souche de *Leuconostoc mesenteroides ssp. cremoris* capable de synthétiser du dextrane n'a été isolée. Or, *Leuconostoc mesenteroides ssp. cremoris* présente une importance majeur dans la fabrication de compositions laitières, telles que les spécialités fermentées type yogourt ou les crèmes laitières, par exemple. Il serait donc très intéressant de pouvoir utiliser de telles bactéries capables de synthétiser du dextrane, présentant une texture et un goût agréables, de manière à texturer notamment ce type de compositions alimentaires.

La présente invention a pour but de répondre à ce besoin.

### RESUME DE L'INVENTION

A cet effet, la présente invention a pour objet une souche de *Leuconostoc mesenteroides ssp. cremoris* produisant du dextrane, notamment les souches de *Leuconostoc mesenteroides ssp. cremoris* CNCM I-1692 et CNCM I-1693.

La présente invention a également pour objet un procédé d'utilisation d'une souche de *Leuconostoc mesenteroides ssp. cremoris* pour la fabrication d'une composition alimentaire ou cosmétique.

Un autre objet de la présente invention est un procédé de production de dextrane de *Leuconostoc mesenteroides ssp. cremoris.*

La présente invention concerne également une composition alimentaire ou cosmétique, à laquelle on incorpore au cours de sa préparation du dextrane obtenu par la mise en oeuvre de ce procédé.

De plus, la présente invention a pour objet un procédé de production d'un additif contenant de la dextrane-sucrase active de *Leuconostoc mesenteroides ssp. cremoris*.

Enfin, la présente invention concerne une composition alimentaire ou cosmétique, à laquelle on incorpore au cours de sa préparation un additif contenant de la dextrane-sucrase active de *Leuconostoc mesenteroides ssp. cremoris.*

### DESCRIPTION DETAILLEE DE L'INVENTION

La présente invention a donc pour objet une souche de *Leuconostoc mesenteroides ssp. cremoris* produisant du dextrane. On a pu isoler des souches de *Leuconostoc mesenteroides ssp. cremoris* produisant du dextrane. Aussi, toutes les souches de *Leuconostoc mesenteroides ssp. cremoris* produisant du dextrane sont comprises dans la présente invention.

On a isolé notamment d'une crème suisse une souche de *Leuconostoc mesenteroides ssp. cremoris* et on a constaté avec surprise qu'elle présente la propriété remarquable de synthétiser du dextrane à la texture et au goût agréables. Cette souche a été déposée le 18/04/96, selon le traité de Budapest, à la Collection Nationale de Cultures de Microorganismes, INSTITUT PASTEUR, 25, rue du Docteur Roux, F-75724 PARIS CEDEX 15 où elle a reçu le numero de dépôt CNCM I-1692.

De plus, on a isolé, par sélection naturelle à partir de la souche CNCM I-1692, une souche de *Leuconostoc mesenteroides ssp. cremoris* qui présente également la propriété remarquable de synthétiser du dextrane à la texture et au goût agréables. Cette souche a été déposée le 18/04/96, selon le traité de Budapest, à la Collection Nationale de Cultures de Microorganismes, INSTITUT PASTEUR, 25, rue du Docteur Roux, F-75724 PARIS CEDEX 15 où elle a reçu le numéro de dépôt CNCM I-1693.

Des détails sur ces souches concernant notamment leur morphologie, la fermentation des sucres et autres sont donnés ci-après.

### Morphologie

- Microorganismes Gram positif,
- Catalase négative,
- Aérobie facultative,
- Cocci

### Fermentation des sucres

- Pas de production d'acide lactique à partir des pentoses, D et L-arabinose, D et L-xylose et D et L-ribose.
- Production d'acide lactique à partir du lactose par la souche CNCM I-1692.
- Pas de production d'acide lactique à partir du lactose par la souche CNCM I-1693.

### Autres

- Souches synthétisant du dextrane, polysaccharide ayant des propriétés texturantes remarquables.

Selon la présente invention, on peut donc isoler également une souche de *Leuconostoc mesenteroides ssp. cremoris* ne fermentant pas le lactose, telle que CNCM I-1693, par exemple.

Une souche préférée selon la présente invention produit le même dextrane que la souche CNCM I-1692 ou que la souche CNCM I-1693.

La présente invention a également pour objet un procédé de production de dextrane, dans lequel on inocule avec une préculture d'une souche de *Leuconostoc mesenteroides ssp. cremoris* selon l'invention un milieu contenant du saccharose, on laisse fermenter à 25-35° C pendant 10-20 h, puis on abaisse le pH de la culture ainsi obtenue à 5-5,5 avant de la stocker à 0-10° C pendant 16-48 h.

On peut inoculer avec une préculture d'une souche de *Leuconostoc mesenteroides ssp. cremoris* selon l'invention un milieu contenant au moins 2% de saccharose, de manière à permettre la production de dextrane-sucrase et la synthèse de dextrane dans le milieu de culture, par exemple.

On peut inoculer avec 0,2-3% d'une préculture d'une souche de *Leuconostoc mesenteroides ssp. cremoris* selon la présente invention, en particulier à partir de la souche CNCM I-1692 ou de la souche CNCM I-1693, un milieu contenant, 5-12% de milieu MSK (lait de vache écrémé) supplémenté avec 0,05-0,2% d'extrait de levure et avec au moins 2% de saccharose, par exemple. On peut laisser fermenter à 25-35° C pendant 10-20 h, tout en maintenant le pH à 6-7,3, par exemple. Puis, en fin de fermentation, on peut abaisser le pH de la culture ainsi obtenue à 5-5,5 par addition d'acide lactique, par exemple. Puis on stocke donc la culture à 0-10° C pendant 16-48 h.

On peut également inoculer avec 0,2-3% d'une préculture d'une souche de *Leuconostoc mesenteroides ssp. cremoris* selon la présente invention, en particulier à partir de la souche CNCM I-1692 ou de la souche CNCM I-1693, un milieu contenant 0,05-0,2% d'extrait de levure, avec au moins 2% de saccharose, par exemple. On peut laisser fermenter à 25-35° C pendant 10-20 h, tout en maintenant le pH à 6-7,3, par exemple. En fin de fermentation, on peut mélanger cette culture volume à volume avec du milieu MSK à 20%, de manière à éviter, au cours de la fermentation, l'inhibition de la production de l'enzyme dextrane-sucrase par le lactose contenu dans le milieu MSK, par exemple. On peut alors abaisser le pH de la culture ainsi obtenue à 5-5,5 par addition d'acide lactique, par exemple. Puis on stocke donc la culture à 0-10° C pendant 16-48 h.

On peut alors sécher cette culture, de manière à obtenir une poudre de dextrane, par exemple. On peut sécher cette culture par lyophilisation ou par séchage par atomisation, par exemple.

La présente invention a également pour objet une composition alimentaire ou cosmétique comprenant du dextrane de *Leuconostoc mesenteroides ssp. cremoris* susceptible d'être obtenu par la mise en oeuvre du procédé ci-dessus.

Pour préparer une telle composition, on peut incorporer du dextrane ainsi obtenu à un produit alimentaire ou cosmétique, tel qu'une poudre de lait, un yogourt, une sauce ketchup, une mayonnaise ou une créme pour la peau, au cours de sa fabrication, par exemple.

La présente invention concerne également un procédé de production d'un additif contenant de la dextrane-sucrase active, dans lequel on inocule avec une préculture d'une souche de *Leuconostoc mesenteroides ssp. cremoris* selon l'invention un milieu contenant du saccharose, puis on laisse fermenter à 25-35° C pendant 10-20 h.

On peut inoculer avec une préculture d'une souche de *Leuconostoc mesenteroides ssp. cremoris* selon l'invention un milieu contenant au moins 2% de saccharose, de manière à permettre la production de dextrane-sucrase dans le milieu de culture, par exemple.

On peut inoculer avec 0,2-3% d'une préculture d'une souche de *Leuconostoc mesenteroides ssp. cremoris* selon la présente invention, en particulier avec 0,2-3% d'une préculture de la souche CNCM I 1692 ou de la souche CNCM I-1693, un milieu contenant 5-12% de milieu MSK (lait de vache écrémé) supplémenté avec 0,05-0,2% d'extrait de levure et avec au moins 2% de saccharose, par exemple. On peut laisser fermenter à 25-35° C pendant 10-20 h, tout en maintenant le pH à 6-7,3, par exemple.

On peut inoculer avec 0,2-3% d'une préculture d'une souche de *Leuconostoc mesenteroides ssp. cremoris* selon la présente invention, en particulier avec 0,2-3% d'une préculture de la souche CNCM I 1692 ou de la souche CNCM I-1693, un milieu contenant 0,05-0,2% d'extrait de levure et au moins 2% de saccharose, par exemple. On peut laisser fermenter à 25-35° C pendant 10-20 h, tout en maintenant le pH à 6-7,3, par exemple. En fin de fermentation, on peut mélanger cette culture volume à volume avec du milieu MSK à 20%, de manière à éviter, au cours de la fermentation, l'inhibition de la production de l'enzyme dextrane-sucrase par le lactose contenu dans le milieu MSK, par exemple

On peut également inoculer avec 0,2-3% d'une préculture d'une souche de *Leuconostoc mesenteroides ssp. cremoris* selon la présente invention, en particulier avec 0,2-3% d'une préculture de la souche CNCM I-1692 ou de la souche CNCM I-1693, un milieu de culture synthétique contenant au moins 2% de saccharose, 1-3% de K₂HPO₄, 0,2-1% d'extrait de levure, 0,2-1% de peptone, 0,0005-0,001% MnSO₄, . On peut laisser fermenter à 25-35° C pendant 7-12 h, tout en maintenant le pH à 6-7,3, par exemple.

Dans un premier mode de réalisation préféré du procédé de préparation d'un additif contenant de la dextrane-sucrase active, après la fermentation, on ajuste le pH de la culture à 5-5,5, puis on sèche cette culture, de manière à obtenir une poudre contenant la dextrane-sucrase active. On peut ajuster le pH de la culture par addition d'acide lactique, par exemple.On peut sécher la culture par lyophilisation ou par séchage par atomisation, par exemple.

Dans un second mode de réalisation préféré du procédé de préparation de la dextrane-sucrase active, on réalise une séparation sur la culture après la fermentation, de manière à isoler le surnageant contenant la dextrane-sucrase active. On peut réaliser cette séparation par centrifugation à 15000-20000 g pendant 10-35 min à 2-6° C, par exemple.

On peut ajuster le pH du surnageant à 4,9-5,7, stocker ce surnageant à 0-10° C pendant 15-30 h, puis précipiter les macromolécules contenues dans ce surnageant, de manière à isoler un précipité contenant la dextrane-sucrase, par exemple. On peut précipiter les macromolécules du surnageant avec du polyéthylèneglycol ou de l'ammonium sulfate à 2-6° C sous agitation, par exemple.

On peut ensuite effectuer une dialyse du précipité contenant la dextrane-sucrase, de manière à écarter les agents de précipitation, par exemple.

Enfin, dans ce second mode de réalisation préféré du présent procédé, on peut stocker le précipité contenant la dextrane-sucrase à une température inférieure à -4° C après l'étape de précipitation ou après l'étape de dialyse, par exemple.

La présente invention a également pour objet une composition alimentaire ou cosmétique, comprenant un additif contenant de la dextrane-sucrase active de *Leuconostoc mesenteroides ssp. cremoris* susceptible d'être obtenu par la mise en oeuvre du procédé ci-dessus.

Pour préparer une telle composition, on peut incorporer un additif contenant de la dextranesucrase active ainsi obtenue à un produit alimentaire ou cosmétique, tel qu'une poudre de lait, un yogourts, une sauce ketchup, une mayonnaise ou une créme pour la peau, au cours de sa fabrication, par exemple.

Enfin, la présente invention a également pour objet un procédé d'utilisation d'une souche de *Leuconostoc mesenteroides ssp. cremoris* selon la présente invention, pour la fabrication d'une composition alimentaire ou cosmétique.

Les souches de *Leuconostoc mesenteroides ssp. cremoris,* la dextrane-sucrase produite par ces souches et le dextrane synthétisé par ces souches selon la présente invention sont caractérisés plus en détails ci-après à l'aide de différentes données microbiologiques et biochimiques illustrant leurs propriétés. Les pourcentages sont donnés en poids sauf indication contraire.

### Recherche des souches de Leuconostocs produisant du dextrane

A partir de 150 souches isolées soit de produits laitiers, soit de produits non laitiers, tels que le vin' le café et la choucroute, on a recherché les souches produisant du dextrane.

On mesure la production de dextrane dans un milieu contenant du saccharose.

Pour ce faire, on inocule avec 1% de préculture de chacune des 150 souches 10 ml de milieu DEX comprenant 1% de B.tryptone, 0,5% d'extrait de levure, 0,5% de K₂HPO₄, 0,5% de citrate d'ammonium et 5% de saccharose. Puis, on laisse fermenter à 30° C pendant 24 h.

On a ainsi selectionné à partir des 150 souches de départ, 16 souches capables de produire du dextrane.

On inocule alors avec 1% de préculture de chacune de ces 16 souches sélectionnées 150 ml de milieu DEX, tel que décrit ci-dessus, puis on laisse fermenter à 30° C pendant 24 h, avant de mesurer la viscosité du produit de ces 16 cultures ainsi obtenues. On mesure la viscosité, à l'aide d'un viscomètre à écoulement de 25 mm de diamètre.

Le tableau I ci-après présente les valeurs en secondes pour faire passer dans le viscomètre à écoulement 100 ml de produit de chaque culture. La souche A1, présentée dans le tableau I, a été utilisée comme souche témoin négatif.

Dans ce tableau, les souches A sont des souches de *Leuconostocs sp.,* les souches B sont des souches de *Leuconostoc mesenteroides ssp. cremoris*, la souche C1 est une souche de *Leuconostoc lactis*, les souches D sont des souches de *Leuconostoc mesenteroides ssp. mesenteroides.*

**Tableau I**

| Souche | Viscosité (s) |
|---|---|
| A1 | 11 |
| A2 | 16 |
| A3 | 14 |
| A4 | 13 |
| A5 | 18 |
| A6 | 20 |
| A7 | 19 |
| A8 | 19 |
| C1 | 17 |
| CNCM I-1692 | 22 |
| B1 | 12 |
| B2 | 14 |
| B3 | 15 |
| D1 | 12 |
| D2 | 14 |
| D3 | 15 |
| D4 | 17 |

Les résultats présentés dans le tableau I ci-dessus mettent en évidence le fait que la souche *Leuconostoc mesenteroides ssp. cremoris* CNCM I-1692 présente la viscosité la plus élevée des 16 souches séléctionnées. On admet donc que la souche de *Leuconostoc mesenteroides ssp. cremoris* CNCM I-1692 produit le plus de dextrane.

### Etude de la concentration de l'enzyme dextrane-sucrase en fonction du temps de fermentation

On inocule avec 1% de préculture de la souche de *Leuconostoc mesenteroides ssp. cremoris* CNCM I-1692 un milieu synthétique contenant 2% de saccharose, 2% de K₂HPO₄, 0,5% d'extrait de levure, 0,5% de peptone, 0,02% de MgSO₄, 0,001% de MnSO₄, 0,001% de FeSO₄ et 0,001% de NaCl. On laisse fermenter à 30° C pendant 12 h dans un fermenteur de 5l.

Après 4 h de fermentation, on prélève toutes les 2 heures, jusqu'à 12 h de fermentation, un échantillon de la culture ainsi obtenue, de manière à mesurer la croissance de la souche par densité optique à 600 nm.

Puis, on centrifuge chaque échantillon à 18000 g pendant 20 min à 4° C, on récupère le surnageant, on ajuste son pH à 5,2 et l'on vérifie l'activité de la dextrane-sucrase, contenue dans le surnageant, par mesure de l'incorporation de radioactivité dans le dextran à partir de saccharose radioactif (J. Dent. Res. 1974, 53, 1355-1360).

Le tableau II ci-après présente les résultats obtenus pour la mesure de la croissance de la souche à partir d'échantillons, prélevés toutes les 2 heures, entre 4 et 12 h de fermentation.
De plus, le tableau II présente les résultats de la mesure de l'activité de la dextrane-sucrase contenue dans le surnageant de ces échantillons.

**Tableau II**

| durée de la fermentation (h) | 4 | 6 | 8 | 10 | 12 |
|---|---|---|---|---|---|
| croissance (DO₆₀₀)/10 | 0,034 | 0,085 | 0,21 | 0,51 | 0,53 |
| activité (u/ml) | - | 0,1 | 0,17 | 0,6 | 0,61 |

Les résultats mentionnés au tableau II mettent en évidence le fait que la concentration en dextrane-sucrase atteint sa valeur maximale juste après l'entrée des bactéries *Leuconostoc mesenteroides ssp. cremoris* en phase stationnaire.

### Purification de l'enzyme dextrane-sucrase et détermination de son activité spécifique

On inocule avec 1% de préculture de *Leuconostoc mesenteroides ssp. cremoris* CNCM I-1692 un milieu de culture contenant 0,2% de saccharose, 0,5% d'extrait de levure, 0,5% de peptone, 2% de K₂HPO₄, 0,02% de MgSO₄, 0,001% de MnSO₄, 0,001% de FeSO₄ et 0,001% de NaCl et on laisse fermenter à température ambiante pendant 12h tout en maintenant le pH à une valeur de 6,7.

Puis, on centrifuge la culture ainsi préparée à 18000 g pendant 20 min à 4° C.

On isole alors le surnageant contenant la dextrane-sucrase et on ajuste son pH à une valeur de 5,2, avant de l'incuber pendant 4h à 4° C.

On mélange ensuite volume à volume le surnageant avec 33% de polyéthylène glycol 400 et on laisse incuber à 4° C pendant 5h sous agitation, de manière à précipiter les protéines contenues dans le surnageant.

On effecute une centrifugation à 18000 g epndant 20 min à 4° C, de manière à isoler le culot contenant les protéines précipitées.

On suspend alors ce culot contenant les protéines précipitées dans 70 ml d'acétate d'ammonium 20 mM, pH 5,2.

On ajoute alors à cette suspension 600 µg de dextranase et on laisse incuber le tout à 25° C pendant 1h, de manière à digérer le dextran contenu dans la suspension par la dextranase.

On effectue une dialyse de la suspension en présence de 70 ml d'acétate d'ammonium 20 mM, pH 5,2, de manière à éliminer les molécules de glucose obtenues, après digestion du dextran avec la dextranase. Puis on isole les protéines sur une colonne échangeur d'anions (Fast Q, Pharmacia Biotech AB, Uppsala, SU) que l'on a préalablement équilibrée avec un tampon d'acétate d'ammonium 20mM, pH 5,2.

Les protéines sont éluées sur un gradient linéaire de NaCl 0-0,5 M.

On analyse l'activité de la dextrane-sucrase des différentes fractions protéiques ainsi éluées et l'on effectue une électrophorèse de ces fractions protéiques sur un gel SDS de polyacrylamide.

On isole les fractions protéiques contenant la dextrane-sucrase et l'on mesure l'activité de la dextrane-sucrase purifiée qui est de 105 u/mg.

### Capacité texturante des dextranes en fonction de la température d'incubation de l'enzyme

On met en évidence l'activité de la dextrane-sucrase de la souche de *Leuconostoc mesenteroides ssp. cremoris* CNCM I-1692 d'une part à 30° C et à d'autre part à 4° C et l'on vérifie ainsi la capacité texturante des dextranes à ces différentes températures.

Pour ce faire, on prépare un additif contenant de la dextrane-sucrase active à partir d'une culture de la souche de *Leuconostoc mesenteroides ssp. cremoris* CNCM I-1692.

On inocule avec 1% de préculture de la souche de *Leuconostoc mesenteroides ssp. cremoris* CNCM I-1692 un milieu synthétique contenant 2% de saccharose, 2% de K₂HPO₄, 0,5% d'extrait de levure, 0,5% de peptone, 0,02% de MgSO₄, 0,001% MnSO₄, 0,001% de FeSO₄ et 0,001% de NaCl. On laisse fermenter à 30° C pendant 12 h, tout en maintenant le pH à 6,7.

Puis, on réalise une séparation sur la culture par centrifugation à 18000 g pendant 20 min à 4° C, de manière à isoler le surnageant contenant la dextrane-sucrase.

On abaisse le pH du surnageant à 5,2 et on incube ce surnageant pendant 12 h à 4° C.

On précipite alors 2 fois au polyéthylèneglycol à 4° C les macromolécules du surnageant, de manière à purifier la dextrane-sucrase.

Puis, on vérifie la capacité texturante des dextranes synthétisés par la dextrane-sucrase ainsi isolée, en incubant cette dernière d'une part à 30° C et d'autre part à 4° C dans un tampon-substrat contenant 20 mM d'acétate pH 5,2, 200 mM de saccharose et 20 mM de CaCl₂.

Le tableau IV ci-après présente les résultats de la texturation avec la dextrane-sucrase active de la souche de *Leuconostoc mesenteroides ssp. cremoris* CNCM I-1692 d'une part à 30° C et d'autre part 4° C.

**Tableau IV**

| **milieu** | **conditions d'incubation** | **texturation** |
|---|---|---|
| a | 30° C pendant 12 h | - |
| | 4° C pendant 12 h | +++++ |
| a: milieu tampon-substrat à pH 5,2 -: absence de texture, +++++: bonne texture, très épaisse. | | |

Les résultats présentés dans le tableau IV mettent en évidence le fait que la dextrane-sucrase de la souche de *Leuconostoc mesenteroides ssp. cremoris* CNCM I-1692 permet de synthétiser à 4° C un dextrane ayant une texture agréable et épaisse, alors qu'à 30° C, on obtient une solution turbide ne présentant aucune texture. Cette différence de texturation est sans doute due au fait que le dextrane à 4° C est sous forme de molécules avec des branches courtes et permet ainsi d'obtenir une texture agréable et épaisse, alors qu'à 30° C, le dextrane se présente sous forme de molécules ayant des branches longues alignées en parallèles avec la chaine principale. Ces molécules ne permettent pas d'obtenir une bonne texture.

### Texturation d'un produit laitier avec la dextrane-sucrase

On incube la dextrane-sucrase dans des conditions (milieu, température et pH) identiques à celles qui prévalent lors de la de préparation d'un yogourt.

Pour ce faire, on prépare un additif contenant de la dextrane-sucrase active à partir d'une culture de la souche de *Leuconostoc mesenteroides ssp. cremoris* CNCM I-1692.

On inocule avec 1% de préculture de la souche de *Leuconostoc mesenteroides ssp. cremoris* CNCM I-1692 un milieu synthétique contenant 2% de saccharose, 2% de K₂HPO₄, 0,5% d'extrait de levure, 0,5% de peptone, 0,02% de MgSO₄, 0,001% de MnSO₄, 0,001% de FeSO₄ et 0,001% de NaCl. On laisse fermenter à 30° C pendant une nuit, tout en maintenant le pH à 6,7.

Puis, on réalise une séparation sur la culture par centrifugation à 18000 g pendant 20 min à 4° C, de manière à isoler le surnageant contenant la dextrane-sucrase.

on abaisse alors le pH du surnageant à 5,2 et l'on incube ce surnageant pendant 12 h à 4° C.

On précipite alors 2 fois au polyéthylèneglycol à 4° C les macromolécules du surnageant, de manière à purifier la dextrane-sucrase.

On vérifie alors la synthèse de dextrane par la dextrane-sucrase. Pour ce faire, on incube la dextrane-sucrase d'une part dans un tampon-substrat à pH 6,4 contenant 20 mM d'acétate 200 mM de saccharose et 20 mM de CaCl₂ et d'autre part dans un lait-drink contenant 6% de saccharose.

Le tableau V ci-après présente les résultats obtenus pour la texturation avec la dextrane-sucrase de la souche de *Leuconostoc mesenteroides ssp. cremoris* CNCM I-1692.

**Tableau V**

| **milieu** | **conditions de simulation** | **texture** |
|---|---|---|
| a | c | - |
| | d | +++++ |
| b | c | + |
| | d | +++++ |
| a: tampon-substrat pH 6,4. b: lait drink contenant 6% de saccharose. c: 5h à 37° C, puis on ajuste le pH à 4,7, on abaisse la température à 4° C et l'on incube 24 h à 4° C. d: 5h à 20° C, puis on ajuste le pH à 4,7, on abaisse la température à 4° C et l'on incube 24 h à 4° C. -: absence de texture. +: peu de texture. +++++: bonne texture. | | |

Les résultats présentés dans le tableau V permettent de mettre en évidence le fait que la dextrane-sucrase texture aussi un milieu laitier, mais ne fait plus de dextrane épaississant après 5 h à 37° C. Pour texturer des yogourts, on peut ajouter la dextrane-sucrase après la fermentation, avant le stockage à 4° C.

Les exemples ci-après sont présentés à titre d'illustration de l'utilisation du dextrane, de la dextrane-sucrase et/ou d'une souche productrice de ce dextrane et de cette dextrane-sucrase dans la fabrication d'une composition alimentaire ou cosmétique selon la présente invention. Les pourcentages y sont donnés en poids sauf indication contraire.

### Exemple 1

On utilise la souche de *Leuconostoc mesenteroides ssp. cremoris* CNCM I-1692 selon la présente invention pour la fabrication de yogourts.

Pour ce faire, on prépare 1l d'une composition laitière contenant 2,8% de graisses, additionnée de 2% de lait écrémé en poudre et de 6% de saccharose, on la pasteurise à 96° C pendant 30 min puis on abaisse sa température à 42° C.

Parallèlement, on réactive une préculture congelée d'une souche de *Streptococus thermophilus* non épaississante et préculture congelée d'une souche de *Lactobacillus bulgaricus* non filante dans un milieu de culture MSK stérile, contenant 10% de poudre de lait reconstituée et 0,1% d'extrait de levure commerciale.

On réactive également une préculture congelée de la souche de *Leuconostoc mesenteroides ssp. cremoris* dans un milieu de culture MRS (lactobacilli MRS - Detroit - USA), puis dans un milieu de culture MSK stérile, contenant 10% de poudre de lait reconstituée et 0,1% d'extrait de levure commerciale, additionné de 1% de saccharose.

On inocule alors la composition laitière pasteurisée avec 1% de chacune de ces précultures réactivées, puis on laisse fermenter à 37° C jusqu'à ce que le pH de cette composition laitière atteigne la valeur de 4,5.

On réalise ainsi des yogourts que l'on conserve à 4° C.

Ces yogourts préparés avec une souche de *leuconostoc mesenteroides ssp. cremoris* présentent une texture onctueuse au goût agréable, surtout après 10 jours de conservation à 4° C.

### Exemple 2

On utilise l'additif contenant de la dextrane-sucrase active selon la présente invention pour la fabrication de yogourts.

Pour ce faire, on inocule avec 1% de préculture de la souche de *Leuconostoc mesenteroides ssp. cremoris* CNCM I-1692 un milieu synthétique contenant 2% de saccharose, 2% de K₂HPO₄, 0,5% d'extrait de levure, 0,5% de peptone, 0,02% de MgSO₄, 0,001% MnSO₄, 0,001% de FeSO₄ et 0,001% de NaCl. On laisse fermenter à 30° C pendant 12 h, tout en maintenant le pH à 6,7. Puis, on réalise une séparation sur la culture par centrifugation à 18000 g pendant 20 min à 4° C, de manière à isoler le surnageant contenant la dextrane-sucrase active.On abaisse le pH du surnageant à 5,2 et on précipite alors les macromolécules du surnageant à 4° C avec de l'ammonium sulfate, de manière à isoler un précipité contenant la dextrane-sucrase. On dialyse alors ce précipité, de manière à éliminer l'ammonium sulfate.

Par ailleurs, on prépare 1l d'une composition laitière contenant 2,8% de graisses, additionnée de 2% de lait ecrémé en poudre et de 6% de saccharose, on la pasteurise à 96° C pendant 30 min puis on abaisse sa température à 42° C.

Parallèlement, on réactive une préculture congelée d'une souche de *Streptococus thermophilus* non épaississante et une préculture congelée d'une souche de *Lactobacillus bulgaricus* non filante dans un milieu de culture MSK stérile, contenant 10% de poudre de lait reconstitué et 0,1% d'extrait de levure commerciale.

On inocule la composition laitière pasteurisée avec 1% de chacune des cultures des deux souches, puis on l'incube à 37° C jusqu'à ce que le pH de la composition laitière atteigne la valeur de 4,5.

Puis, on ajoute sous agitation 1‰ de dextrane-sucrase purifiée.

On réalise ainsi des yogourts que l'on conserve à 4° C.

Ces yogourts préparés avec la dextrane-sucrase purifiée, synthétisée par *Leuconostoc mesenteroides ssp. cremoris* présentent une texture onctueuse au goût agréable, surtout après 10 jours de conservation à 4° C.

### Exemple 3

On utilise, sous forme de poudre, l'additif contenant de la dextrane-sucrase active selon la présente invention, pour la fabrication de yogourts.

Pour ce faire, on inocule avec 1% de préculture de la souche de *Leuconostoc mesenteroides ssp. cremoris* un milieu de culture contenant 9% de lait écrémé en poudre, 0,1% d'extrait de levure, et 2% de saccharose. On laisse fermenter à 30° C pendant 20 h tout en maintenant le pH à 6,7. On mélange la culture volume à volume avec une solution de MSK à 20%. Puis, on abaisse le pH de la culture ainsi obtenue à 5,2 par addition d'acide lactique, avant de la sécher par atomisation, de manière à obtenir l'additif contenant de la dextrane-sucrase active sous forme de poudre.

Par ailleurs, on prépare 1l d'une composition laitière contenant 2,8% de graisses, additionnée de 2% de lait ecrémé en poudre et de 6% de saccharose, on la pasteurise à 96° C pendant 30 min puis on abaisse sa température à 42° C.

Parallèlement, on réactive une préculture congelée d'une souche de *Streptococus thermophilus* non épaississante et une préculture congelée d'une souche de *Lactobacillus bulgaricus* non filante dans un milieu de culture MSK stérile, contenant 10% de poudre de lait reconstitué et 0,1% d'extrait de levure commerciale.

On inocule la composition laitière pasteurisée avec 1% de chacune des cultures des deux souches, puis on l'incube à 40° C jusqu'à ce que le pH de la composition laitière atteigne la valeur de 4,5. Puis, on ajoute sous agitation 1% de l'additif, sous forme de poudre, contenant la dextrane-sucrase active.

On réalise ainsi des yogourts que l'on conserve à 4° C.

Ces yogourts préparés avec l'additif contenant de la dextrane-sucrase active, sous forme de poudre, présentent une texture onctueuse au goût agréable, surtout après 10 jours de conservation à 4° C.

## Revendications

**1.** Souche de *Leuconostoc mesenteroides ssp. cremoris* produisant du dextrane.

**2.** Souche selon la revendication 1, déposée à la CNCM sous le numéro I-1692.

**3.** Souche selon la revendication 1, déposée à la CNCM sous le numéro I-1693.

**4.** Souche selon la revendication 1, produisant le même dextrane que la souche CNCM I-1692 ou que la souche CNCM I-1693.

**5.** Souche selon l'une des revendications 1,3 et 4, ne fermentant pas le lactose.

**6.** Procédé de production de dextrane, dans lequel:
- on inocule avec une préculture d'une souche selon l'une des revendications 1-5 un milieu de culture contenant du saccharose,
- on laisse fermenter à 25-35° C pendant 10-20 h,
- puis on abaisse le pH de la culture ainsi obtenue à 5-5,5, avant de la stocker à 0-10° C pendant 16-48 h.

**7.** Procédé selon la revendication 6, dans lequel le milieu de culture contient au moins 2% de saccharose.

**8.** Procédé selon la revendication 6, dans lequel on mélange la culture à un milieu laitier juste après l'étape de fermentation.

**8.** Procédé selon la revendication 6, dans lequel on sèche la culture après l'étape de stockage.

**9.** Composition alimentaire ou cosmétique, comprenant du dextrane de *Leuconostoc mesenteroides ssp. cremoris* susceptible d'être obtenu par la mise en oeuvre du procédé selon l'une des revendications 6-8.

**10.** Procédé de production d'un additif contenant de la dextrane-sucrase active dans lequel on inocule avec une préculture d'une souche selon l'une des revendications 1-5 un milieu contenant du saccharose, puis on laisse fermenter à 25-35° C pendant 10-20 h.

**11.** Procédé selon la revendication 10, dans lequel le milieu de culture contient au moins 2% de saccharose.

**12.** Procédé selon la revendication 10, dans lequel, après la fermentation, on ajuste le pH de la culture à 5-5,5 puis on sèche cette culture.

**13.** Procédé selon la revendication 10, dans lequel on réalise une séparation sur la culture après la fermentation, de manière à isoler le surnageant contenant la dextrane-sucrase.

**14.** Procédé selon la revendication 13, dans lequel on ajuste le pH du surnageant à 4,9-5,7, on le stocke à 0-10° C pendant 15-30 h, puis on précipite les macromolécules contenues dans le surnageant, de manière à isoler un précipité contenant la dextrane-sucrase.

**15.** Procédé selon la revendication 14, dans lequel on effectue une dialyse du précipité contenant la dextrane-sucrase.

**16.** Procédé selon l'une des revendications 14-15, dans lequel on stocke le précipité contenant la dextrane-sucrase à une température inférieure à -4° C.

**17.** Composition alimentaire ou cosmétique, comprenant un additif contenant de la dextrane-sucrase active de *Leuconostoc mesenteroides ssp. cremoris* susceptible d'être obtenu par la mise en oeuvre du procédé selon l'une des revendications 10 à 16.

**18.** Procédé d'utilisation d'une souche selon l'une des revendications 1-5, pour la fabrication d'une composition alimentaire ou cosmétique.
